(19) Europäisches Patentamt
European Patent Office
Office européen des brevets

(11) Veröffentlichungsnummer: **0 131 615**
**B1**

(12) **EUROPÄISCHE PATENTSCHRIFT**

(45) Veröffentlichungstag der Patentschrift: **20.05.87**

(51) Int. Cl.⁴: **A 61 B 5/02,** A 61 B 5/08

(21) Anmeldenummer: **84900559.0**

(22) Anmeldetag: **19.01.84**

(86) Internationale Anmeldenummer: **PCT/SE 84/00018**

(87) Internationale Veröffentlichungsnummer: **WO 84/02837 (02.08.84 Gazette 84/18)**

(54) **ANORDNUNG ZUR BESTIMMUNG DES MINUTENVOLUMENS DES HERZENS.**

(30) Priorität: **19.01.83 SE 8300246**

(43) Veröffentlichungstag der Anmeldung: **23.01.85 Patentblatt 85/4**

(45) Bekanntmachung des Hinweises auf die Patenterteilung: **20.05.87 Patentblatt 87/21**

(84) Benannte Vertragsstaaten: **CH DE FR GB LI**

(56) Entgegenhaltungen: **US-A-4 363 327**

(73) Patentinhaber: **KAROLINSKA INSTITUTET, Institutionen för medicinsk teknik box 60400, S-104 01 Stockholm 60 (SE)**

(72) Erfinder: **LINNARSSON, Dag, Langängsvägen 53, S-182 75 Stocksund (SE)**
Erfinder: **LARSSON, Hans, Högalidsgatan 48 A, S-117 30 Stockholm (SE)**

(74) Vertreter: **Stahl, Gerhard F.W., Patentanwalt Dipl.-Ing. Gerhard F.W. Stahl Heilmannstrasse 10, D-8000 München 71 (DE)**

**EP 0 131 615 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft eine Anordnung zur Bestimmung des Minutenvolumens des Herzens, $\dot{Q}$, einbegriffen die Bestimmung des $CO_2$-Gehaltes, des $N_2O$-Gehaltes oder des Gehaltes eines anderen im Blut ausreichend lösbaren Gases vor und nach dem Durchgang des Blutes durch das Herz, mit Mitteln, um für jeden Atemzug ausgeatmete und eingeatmete Luft zu separieren, einem Mittel zur Messung der Größe des ausgeatmeten Luftstromes und einem Mittel zur Bestimmung des Gasgehaltes des Atmungsstromes.

In dem Buch "Medicin och teknik" von Bertil Jacobson, zweite Auflage, Studentliteratur, Lund 1975, Seite 179-182 sind verschiedene Verfahren zur Bestimmung des Minutenvolumens des Herzens beschrieben. Es handelt sich dabei um das Verfahren nach Fick, das Indikatorverdünnungsverfahren und das Thermodilutionsverfahren. Diese drei Verfahren sind sogenannte invasive Verfahren, d.h. sie umfassen die Einführung eines Katheters in bestimmte Blutgefäße, die Entnahme von Blutproben, Injizierung verschiedener Mittel usw.

Im Gegensatz dazu gibt es die nicht-invasiven oder unblutigen Verfahren, bei denen die ausgeatmete Luft analysiert wird. Bei einer hierzu verwendeten Anordnung der eingangs genannten Gattung ist ein sogenannter Rückatmungssack erforderlich, in den der Patient rückatmet, und aus dem der Patient anschließend wieder einatmet. Die Verwendung eines solchen Rückatmungssackes ist jedoch in konstruktiver, medizinisch/hygienischer und in meßtechnischer Hinsicht unbefriedigend.

Der Erfindung liegt die Aufgabe zugrunde, die gattungsgemäße Anordnung dahingehend weiterzubilden, daß der Rückatmungssack entfällt.

Die Lösung dieser Aufgabe ist im Patentanspruch 1 angegeben.

Die Erfindung wird nachfolgend anhand der Zeichnungen näher erläutert. Es zeigen:

Fig. 1 schematisch den das Herz und die Lunge einschließenden Teil des Blutkreislaufes,

Fig. 2 die gleichen Teile des Blutkreislaufes, wobei jedoch bestimmte Parameter eingetragen sind,

Fig. 3 ein bekanntes Verfahren zur Bestimmung des Minutenvolumens des Herzens,

Fig. 4 einen bei diesem Verfahren durchgeführten zweiten Schritt,

Fig. 5 eine Anordnung nach der Erfindung,

Fig. 6 eine Darstellung ähnlich Fig. 2, und

Fig. 7 eine abgewandelte Anordnung nach der Erfindung.

Das Herz pumpt in Ruhe ca 5 l Blut/Min. (Schlagvolumen ca 75 ml, Schlagfrequenz ca 70 Schläge/Min.). Bei schwerer Muskelarbeit kann das gepumpte Volumen per Minute (normalerweise als das Minutenvolumen bezeichnet), bis auf 25 l/Min. steigen, wobei sich Schlagvolumen und Schlagvolumen und Schlagfrequenz mit einem Faktor 2-3 vergrössern. Normal passiert der Blutstrom in der Folge rechte Herzhälfte 1, Lunge 2 und linke Herzhälfte 3. Das Herzminutenvolumen kann als der Ein- oder Ausfluss des Blutes in einen dieser drei Abschnitte des centralen Kreislaufes definiert werden.

In Fig. 1 wird schematisch von links nach rechts die rechte Herzhälfte 1, die Lunge 2 und die linke Herzhälfte 3 gezeigt. Zum rechten Vorhof leitet die obere und untere Hohlvene H mit $CO_2$ angereichertes Blut und von der rechten Kammer wird dieses über die Lungenarterie L zur Lunge geleitet. In der Lunge wird beim Atmen $CO_2$ abgegeben (obere Pfeilspitze) und $O_2$ wird aufgenommen (untere Pfeilspitze). Mit Oxygen angereichertes Blut geht über die Lungenvenen V zum linken Vorhof und zur linken Kammer in die Aorta A und weiter in den Körper.

Das Herzminutenvolumen ist eine wichtige Variable zu bestimmen bei schwerkranken Patienten z.B. bei Schockzuständen verschiedener Art, grösseren operativen Eingriffen usw. Das Herzminutenvolumen ist auch eine wichtige Variable, zu studieren innerhalb der Arbeitzmedizin, Sportmedizin, der Rehabilitierung oder sonst, wenn man bestimmen will, welche Faktoren die physische Arbeitsfähigkeit beeinflussen.

Konventionell bestimmt man das Herzminutenvolumen mit invasiven (blutigen) Methoden, was die Einführung von Kathetern in die Blutbahn bedeutet. Dies ist mit gewissen Gefahren und Unbehagen für den Patienten verbunden, was in den Fällen, bei denen ohnehin invasive Methoden verwendet werden aufgewogen wird durch den Vorteil, dass das Herzminutenvolumen des Patienten bestimmt werden kann sowie die sich daraus ergebenden Behandlungsmassnahmen. Die invasiven Methoden umfassen normalerweise das Einführen von Kathetern über eine Vene durch die rechte Herzhälfte 1 und zur Lungenpulsader L sowie Injektion einer Indikatorflüssigkeit (Thermodilution), in der Regel gekühlte Kochsalzlösung. Bei einer anderen, mehr komplizierten Methode, werden perifere Arterien für Entnahme von Proben punktiert und ein Farbstoff auf der Venenseite injiziert (Farbverdünnung). Bei der ältesten und meist komplizierten Bestimmungsmethode werden Katheter sowohl in der Lungenpulsader L, als auch in einer periferen Arterie eingesetzt und man entnimmt Proben des Oxygengehaltes in sowohl gemischtem Venenblut als auch in Arterienblut. Gleichzeitig wird die Aufnahme von Oxygen zu den Lungen bestimmt. Diese Bestimmungsmethode folgt Ficks Prinzip und wird in Fig. 2 illustriert:

$\dot{V} = \dot{Q} \cdot C_{\bar{v}} - \dot{Q} \cdot C_a$

$\dot{Q} = \frac{\dot{V}}{C_{\bar{v}} - C_a}$ (Gleichung 1)

Hier ist

$\dot{V}$ = Oxygenaufnahme (ml $O_2$/Min.)

$\dot{Q}$ = Blutstrom durch die Lungen (1 Blut/Min.)

$C_{\bar{v}}$ = Gehalt von Oxygen in gemischtem

Venenblut (ml $O_2$/l Blut)

$C_a$ = Gehalt von Oxygen im Arterienblut (ml $O_2$/l Blut)

Im Prinzip kann die entsprechende Berechnung sowohl auf der Basis von Kohlendioxyd-($CO_2$)-Abgabe der Lunge oder dem Kohlendioxydgehalt des Blutes ausgeführt werden. $\dot{V}$ ist in dem Falle $CO_2$-Abgabe und $C_{\bar{v}}$ bzw. $C_a$ gibt den $CO_2$-Gehalt des Blutes an.

Weiter gibt es so genannte nicht-invasive Methoden zur Bestimmung des Herzminutenvolumens wie schon oben angedeutet wurde. Das Gemeinsame für diese Methoden ist, dass man $\dot{Q}$ auf Grund von $C_{\bar{v}}$ und $C_a$-Werten bestimmt, welche ohne Blutprobeentnahme geschätzt werden. $\dot{V}$ bestimmt man wie immer nicht-invasiv durch Aufsammeln und Analysieren der ausgeatmeten Luft. Danach wird $\dot{Q}$ nach Ficks Prinzip berechnet. Die Schätzung von $C_{\bar{v}}$ und $C_a$ ist mit Fehlerquellen behaftet, die jedoch geringer sind wenn man die Berechnung auf der Basis von $CO_2$-Abgabe an Stelle von $O_2$-Aufnahme ausführt. Der Grund dafür ist, dass $CO_2$ leichter zwischen Lunge und Blut passiert sowie, dass der $CO_2$-Gehalt des Blutes ein ziemlich einfaches Verhältnis zum $CO_2$-Gehalt der Lungenluft besitzt.

Nicht-invasive Bestimmung des Herzminutenvolumens auf Basis des $CO_2$-Austausches zwischen Blut und Lungen ist eine seit Jahrzehnten bekannte Methodik. Es ist eine komplizierte Methode die die Mitwirkung des Patienten sowie umfangreiche Ausrüstung erfordert. Die Prozedur geschieht in zwei Etappen und fordert, dass der Gasaustausch und das Herzminutenvolumen während mehrerer Minuten stabil ist. Dabei wird erst die $CO_2$-Abgabe bestimmt. Die ausgeatmete Spitzenkonzentration von $CO_2$ wird nach Fig. 3 mit einem schnellen $CO_2$-Analysator 4 gemessen. Diese Spitzenkonzentration, die in der Kurve oben links in Fig. 3 gezeigt wird und oft den Mittelwert für mehrere Atemzüge darstellt, wird in Partialdruck ($P_{end\text{-}tidal}$) umgerechnet, dieser Wert ist ungefähr gleich dem Partialdruck von $CO_2$, der im Arterienblut herrscht ($P_a$). Auf Grund eines standardisierten, nichtlinearen Verhältnisses zwischen Partialdruck (P) und Volumengehalt (C) für $CO_2$ im Blut, wird danach $C_a$, d.h. $CO_2$-Gehalt im arterialisierten Blut, das die Lunge verlässt, berechnet. Siehe Diagramm oben rechts in Fig. 3. Danach wird in einer zweiten Etappe der gemischte venöse Gehalt von $CO_2$ bestimmt. Der Patient rückatmet 1-2 l/Atemzug in einen Sack 5 wobei der $CO_2$-Partialdruck im Sack und in der Lunge asymptotisch gegen den gleichen Wert $P_{\bar{v}}$, der im $CO_2$-reichen Blute herrscht und das in die Lunge kommt, steigen wird. Die Rückatmung nimmt etwa 20 Sekunden in Anspruch. Siehe Fig. 4.

Die oben beschriebene Methode ist mit mehreren Nachteilen und Ungelegenheiten behaftet:

- **Praktische**:

Erfordert Mitwirkung des Patienten und komplizierte Apparatur mit grossen mechanischen Ventilen und die exakte Füllung des Rückatmungssackes 5.

- **Medizinische/hygienische**:

Gefahr für Oxygenmangel während der Wiederverwendung auch, wenn initial ein $O_2$-Überschuss im Sack 5 vorhanden ist. Unhygienisch mit Wiederverwendung ausgeatmeten Gases.

- **Theoretisch/berechnungsmässige**:

Die Schätzung von $C_a$ von ausgeatmeter Spitzenkonzentration stützt sich auf mehrere unsichere Annahmen. Die Schätzung von $C_{\bar{v}}$ stützt sich auf Extrapolierung und ist noch mehr unsicher. Die Annahme, dass sämtliches $CO_2$ das das Blut zur Lunge verlässt, gleichzeitig die Lunge zur Umgebung verlässt, ist unsicher; $CO_2$ kann im Lungengewebe gelagert werden.

Der Zweck der Erfindung ist, diese Ungelegenheiten zu eliminieren. Das Verfahren der Erfindung nach umfasst zwei prinzipielle neue Teilmomente, die die Erfindung kennzeichnen.

A. Die Rückatmungsblase wird durch ein elektronisches System ersetzt, das die ausgeatmete $CO_2$-Menge misst und danach die entsprechende $CO_2$-Menge in der Einatmungsluft des darauf folgenden Atemzuges dosiert (Fig. 5). Man erhält so eine selektive Rückatmung von nur einer Gaskomponente und man erhält eine sicherere und einfachere Weise die Etappe in der konventionellen Rückatmungsklinik zu erreichen, die in Fig. 4 beschrieben ist.

Die Anordnung in Fig. 5 umfasst ein Einatmungsrohr 13 und ein Ausatmungsrohr 14. Ein Geber 15 ist mit einer Anordnung 10 zum Messen des $CO_2$-Gehaltes

$$(P_{CO_2})$$

der ausgeatmeten Luft verbunden. Ein anderer Geber 16 ist mit einer Anordnung 8 zur Messung des Ausatmungsstromes verbunden. Eine Quelle 12 für $CO_2$ ist an ein Solenoid 11 angeschlossen, das von einem Mikroprozessor (µP) 6 für die Dosierung von $CO_2$ zum Einatmungsrohr 13 gesteuert wird. Die Anordnungen 8 und 10 steuern den Mikroprozessor 6, der wiederum eine Indikationsanordnung 9 steuert. Ausgeatmetes und eingeatmetes Gas wird von Atmungsventilen separiert. Ausgeatmete Strömung und ausgeatmeter $CO_2$-Gehalt werden direkt im

Ausatmungsstrom von den Gebern 15, 16 gemessen. Der Mikroprozessor 6 (µP) berechnet die ausgeatmete $CO_2$-Menge und steuert eine Dosierung, so dass exakt eine gleich grosse Menge in die Einatmungsluft während des nächst folgenden Atemzuges dosiert wird.

B. Das andere prinzipiell neue Moment in der Erfindung stellt eine neue Berechnungsmethode dar, die durch die Anordnung Fig. 5 nach ermöglicht wird, wenn diese im Hinblick auf die Gasdosierung modifiziert wird.

Das Verfahren gemäss der Erfindung wird dadurch gekennzeichnet, dass die Dosierung des genannten Gases so gesteuert wird, dass der Gasgehalt der Lunge sich nach einem bestimmten Programm ändert (z.B. eine lineare Zunahme im Bezug auf die Zeit, und dass der Wert $\dot{Q}$ bestimmt wird für paarweise Atemzüge als Quote zwischen dem Unterschied zwischen dem effektiven Gasstrom/Zeiteinheit $V_1$ für einen Atemzug und $V_2$ für den nächsten Atemzug und dem Unterschied zwischen dem Gasgehalt $C_{a1}$ bzw. $C_{a2}$ in arteriellem Blut, dass der Wert $\dot{Q}$ für eine Anzahl Atemzugpaare bestimmt wird, zwischen welchen Paaren eine gewisse Anzahl von Atemzügen Zwischenraum ist, sowie dass der Mittelwert der verschiedenen erhaltenen Werten von $\dot{Q}$ berechnet wird.

Die Anordnung gemäss der Erfindung wird im wesentlichen durch einen Mikrocomputer gekennzeichnet, der programmiert ist, dass er die Gasdosierung so steuert, dass sich der Gasgehalt der Lunge nach einem bestimmten Programm ändert, und angeordnet ist, dass er $\dot{Q}$ für paarweise Atemzüge bestimmt nach der Formel

$\dot{Q} = \frac{\dot{V}_1 - \dot{V}_2}{C_{a2} - C_{a1}}$ (Gleichung 4)

worin $\dot{V}$ ist der effektive Gasstrom per Zeiteinheit berechnet Atemzug für Atemzug,

$C_a$ ist der gleichzeitig berechnete Gasgehalt per Einheit arterielles Blutes, wobei Index 1 bezeichnet den ersten Atemzug und Index 2 bezeichnet den darauf folgenden Atemzug wobei der Mikrocomputer $\dot{Q}$ für eine Anzahl Atmungspaare berechnet, welche Paare eine gewisse Anzahl Atemzugzwischenräume haben, und den Mittelwert der auf diese Weise erhaltenen Werte von $\dot{Q}$ bestimmt.

$\dot{V}$ bezeichnet hier den effektiven $CO_2$-Strom per Zeiteinheit, berechnet Atemzug für Atemzug, d.h. (eingeatmete $CO_2$-Menge - ausgeatmete $CO_2$-Menge)/Zeit für den Atemzug. Für einen einzelnen Atemzug erhält man den Wert $V_1$, und eine Massen-Balance-Gleichung für die $CO_2$-Strömungen kann aufgestellt werden:

$C_{\bar{v}} \cdot \dot{Q} = \dot{V}_1 + C_{a1} \cdot \dot{Q} + \Delta$ Speicherung (Gleichung 2)

$C_{a1}$ ist der gleichzeitig berechnete $CO_2$-Gehalt im Arterienblut. Während eines späteren Atemzuges liegt der Wert $V_2$ vor und ein anderer, höherer Wert von $C_a$ ($C_{a2}$). Eine zweite Massen-Balance-Gleichung kann dann aufgestellt werden:

$C_{\bar{v}} \cdot \dot{Q} = \dot{V}_2 + C_{a2} \cdot \dot{Q} + \Delta$ Speicherung (Gleichung 3)

$\dot{Q}$ und $C_{\bar{v}}$ sind während der kurzen Zeit von ca 2 Sekunden die die zwei Atemzüge voneinander trennen, definitionsmässig unverändert. Der Ausdruck "$\Delta$ Speicherung" ist durch die kontinuierliche Zunahme des $CO_2$-Gehaltes der Lunge bedingt. So lange der $CO_2$-Gehalt der Lunge linear zunimmt, ist der Ausdruck "$\Delta$ Speicherung" konstant. Die zwei Gleichungen werden subtrahiert und man erhält einen einfachen mathematischen Ausdruck für die Berechnung von $\dot{Q}$ (Gleichung 4, siehe oben). Die Berechnung wird für 5-6 Paare von Atemzügen, die paarweise mit z.B. 4 Atemzügen Zwischenraum liegen, wiederholt. Danach wird der Mittelwert für $\dot{Q}$ berechnet. Der gesamte Messvorgang wird in einer Etappe während ca 20 Sekunden ausgeführt.

Die obengenannten theoretisch/berechnungsmässigen Nachteile mit der bekannten Technik werden zum grossen Teil eliminiert. Die Schätzung von $C_a$ ist noch immer mit einem unsicheren Fehler behaftet, da jedoch zwei Werte mit dem gleichen Fehler subtrahiert werden, fällt der unsichere Fehler weg. Keine Extrapolierung von $C_{\bar{v}}$ braucht vorgenommen zu werden, da $C_{\bar{v}}$ nicht in der endgültigen Berechnung enthalten ist.

Eine andere Version, die für den gleichen Berechnungsgang wie den in Fig. 5 dargestellten verwendet werden kann, wird in Fig. 7 veranschaulicht und ist etwas mehr kompliziert. Die Anordnung enthält einen Strömungsmesser 18 auch für den Einatmungsstrom sowie ein Mischkammer 7 im Anschluss an die $CO_2$-Dosierung.

Die Dosierung von reinem $CO_2$ kann so mit dem Einatmungsstrom synchronisiert werden, so dass man eine gleichmässige Mischung erhält, auch bei ungleichmässiger Spontanatmung. Die Dosierung von $CO_2$ wird durch eine Gruppe von Magnetventilen zustande gebracht, deren Öffnungsfrequenz und Öffnungszeit durch die zugeleitete $CO_2$-Menge bestimmt wird. Das zugeleitete $CO_2$-Gas kann bei der Dosierung mit $O_2$-gemischt sein, um die Senkung des $O_2$-Gehaltes des eingeatmeten Gases im Zusammenhang mit der $CO_2$-Zufuhr zu vermeiden. Maximal wird 8% $CO_2$ in der Lungenluft erreicht.

Bei der in Fig. 7 gezeigten Variante bezeichnen die Details 6 und 8-16 die gleichen Teile wie in Fig. 5. Ein Strömungsmesser 18 für die Einatmungsluft mit einem Geber 17 kommt hinzu, ausserdem eine Mischkammer 7. Dieser Messer 18 steuert ausser die Anordnungen 8 und 10 den Mikroprozessor 6, der wie schon vorher, die $CO_2$-Dosierung der Einatmungsluft steuert.

Eine weitere Ausführung gleicht in jeder Hinsicht der vorher beschriebenen Ausführung, ausser dass Lachgas ($N_2O$) oder anderes Gas mit ausreichender Lösungsfähigheit im Blut an Stelle von $CO_2$ zugeführt wird. Lachgas in geringeren Konzentrationen beeinflusst das Bewustsein nicht und hat den Vorteil, die Atmung nicht zu stimulieren und ein Gefühl der Atemlosigkeit zu erzeugen auf gleiche Weise wie $CO_2$.

## Patentansprüche

1. Anordnung zur Bestimmung des Minutenvolumens des Herzens, $\dot{Q}$, einbegriffen die Bestimmung des $CO_2$-Gehaltes, des $N_2O$-Gehaltes oder des Gehaltes eines anderen im Blut ausreichend lösbaren Gases vor und nach dem Durchgang des Blutes durch das Herz, mit Mitteln, um für jeden Atemzug ausgeatmete und eingeatmete Luft zu separieren, einem Mittel (8, 16) zur Messung der Größe des ausgeatmeten Luftstromes und einem Mittel (15, 10) zur Bestimmung des Gasgehaltes des Atmungsstromes, gekennzeichnet durch ein Mittel (16, 11), das von den Strömungsmeß- und Gehaltsbestimmungsmitteln (8, 16, 15, 10) gesteuert wird zur dosierten Zuführung des genannten Gases von einer Gasquelle (18) zum Einatmungsstrom während der nächstfolgenden Einatmung und einen Mikrocomputer (6), der dazu programmiert ist, die Gasdosierung so zu steuern, daß der Gehalt des Gases in der Lunge linear zunimmt, und so ausgestaltet ist, daß er Q für paarweise Atemzüge nach folgender Formel bestimmt:

$$\dot{Q} = \frac{\dot{V}_1 - \dot{V}_2}{C_{a1} - C_{a2}}$$

worin $\dot{V}$ die effektive Gasströmung per Zeiteinheit ist, berechnet Atemzug für Atemzug, und $C_a$ der gleichzeitig aufgrund eines standartisierten Verhältnisses zwischen Partialdruck in der ausgeatmeten Luft und Volumengehalt im Blut berechnete Gasgehalt per Einheit arteriellem Blutes ist, wobei der Index 1 einen ersten Atemzug und der Index 2 einen darauf folgenden Atemzug bezeichnet, wobei der Mikrocomputer so ausgestaltet ist, daß er $\dot{Q}$ für eine Anzahl von Atempaaren, deren Paare eine bestimmte Anzahl von Atemzugzwischenräumen haben, berechnet, und den Mittelwert der so gewonnenen verschiedenen Werte von $\dot{Q}$ bestimmt.

2. Anordnung nach Anspruch 1, dadurch gekennzeichnet, daß der Mikrocomputer (6) von einem ersten Geber (15), dessen Ausgangssignal einer Anordnung (10) für die Bestimmung des Gasgehaltes zugeführt wird, von einem zweiten Geber (16), dessen Ausgangssignal einer Anordnung (8) für die Bestimmung des Ausatmungsluftstromes zugeführt wird, sowie von einem dritten Geber (17), dessen Ausgangssignal einer Anordnung (18) für die Bestimmung des Einatmungsluftstromes zugeführt wird, gesteuert wird, wobei der Computer (6) dazu programmiert ist, als Funktion der ausgehenden Signale der genannten Bestimmungsanordnungen (10, 8, 18) Solenoide (11) zu steuern, die als Ventile zwischen einer Gasquelle (12) und einer mit der Einatmungsluft und dem genannten Gas beschickten Mischkammer (7) wirken, und daß der Mikrocomputer (6) einen Indikator (9) für den $\dot{Q}$-Wert steuert.

## Claims

1. Apparatus for the determination of the minute volume of the heart, $\dot{Q}$, including determination of the $CO_2$ content, the $N_2O$ content, or the content of another gas adequately soluble in blood, before and after the passage of the blood through the heart, with means for separating air exhaled and inhaled at each breath, a means (8, 16) for measuring the quantity of the exhaled air flow, and a means (15, 10) for the determination of the gas content of the respiration flow, characterised by a means (16, 11) which is controlled by the flow measurement and content determination means (8, 16, 15, 10) for the dosaged introduction of the aforesaid gas from a gas source (18) into the inhalation flow during the next inhalation in succession, and a microcomputer (6) which is programmed to control the gas dosaging in such a manner that the content of the gas in the lung increases linearly, and is so arranged that it determines $\dot{Q}$ for pairwise breaths in accordance with the following formula:

$$\dot{Q} = \frac{\dot{V}_1 - \dot{V}_2}{C_{a1} - C_{a2}}$$

wherein $\dot{V}$ is the effective gas flow per unit of time, calculated breath by breath, and $C_a$ the gas content per unit of arterial blood calculated at the same time in accordance with a standardised relationship between partial pressure in the exhaled air and volume content in the blood, the index 1 designating a first breath and the index 2 a breath which follows the first, the microcomputer being so arranged that it calculates $\dot{Q}$ for a number of pairs of breaths whose pairs have a specific number of breath intervals, and determines the mean value of the various values of $\dot{Q}$ which are thus obtained.

2. Apparatus according to claim 1, characterised in that the microcomputer (6) is controlled by a first signal emitter (15) whose output signal is fed to an apparatus (10) for the determination of gas content, by a second signal emitter (16) whose output signal is fed to an apparatus (8) for the determination of the exhalation air flow, and also by a third signal emitter (17) whose output signal is fed to an apparatus (18) for the determination of the inhalation air flow, the computer (6) being programmed for controlling, as a function of the outgoing signals from the said determination apparatus (10, 8, 18), solenoids (11) which act as valves between a gas source (12) and a mixing chamber (7) which is fed with the inhalation air and with the aforesaid gas, and that the microcomputer (6) controls an indicator (9) for the $\dot{Q}$ value.

## Revendications

1. Dispositif pour déterminer le débit cardiaque par minute ainsi que la teneur en $CO_2$, $N_2O$ ou d'un autre gaz dissous temporairement dans le

sang avant ou après sa traversée du coeur équipé de moyens pour séparer l'air expiré et inspiré à chaque cycle respiratoire, d'un moyen (8, 16) pour mesurer l'importance du flux d'air expiré et d'un moyen (15, 10) pour évaluer la teneur en gaz du flux respiratoire caractérisé en ce qu'il comprend un moyen (16, 11) qui est commandé par les moyens (8, 16, 15, 10) de mesure du flux et d'évaluation de la teneur pour doser le transfert du gaz choisi d'une source de gaz (18) au flux d'inspiration pendant l'inspiration suivante et un microcalculateur (6) programmé pour commander le dosage en gaz afin que la teneur en gaz des poumons soit linéaire et conformé afin que le débit $\dot{Q}$ d'un couple de cycles satisfasse à la relation

$$\dot{Q} = \frac{\dot{V}_1 - \dot{V}_2}{C_a 1 - C_a 2}$$

où $\dot{V}$ désigne le flux gazeux réel par unité de temps calculé cycle par cycle

$C_a$ est la teneur en gaz simultanée calculée par unité de sang artériel sur la base d'un rapport normalisé entre la pression partielle dans l'air expiré et la teneur volumique du sang, l'indice 1 repérant un premier cycle et l'indice 2 un cycle suivant et soit calculé pour un nombre de cycles dont les couples ont un nombre d'intervalles déterminé et afin que soit déterminée la moyenne des différentes valeurs de $\dot{Q}$ ainsi obtenues.

2. Dispositif suivant la revendication 1, carcatérisé en ce que le microcalculateur (6) est commandé par un premier capteur (15) dont le signal de sortie est acheminé à un agencement (10) pour la détermination de la teneur en gaz, un deuxième capteur (16) dont le signal de sortie est acheminé vers un agencement (8) pour la détermination du flux d'air expiré et un troisième capteur (17) dont le signal de sortie est acheminé vers un agencement (18) pour la détermination du flux d'air inspiré et où ce calculateur (16) est programmé en fonction des signaux émis par les agencements (10, 8, 18) pour commander des relais (11) qui fonctionnement comme des vannes entre une source de gaz (12) et une chambre de mélange (7) recevant l'air inspiré et ce gaz et aussi pour commander un indicateur (9) de la valeur de $\dot{Q}$.

Fig. 1

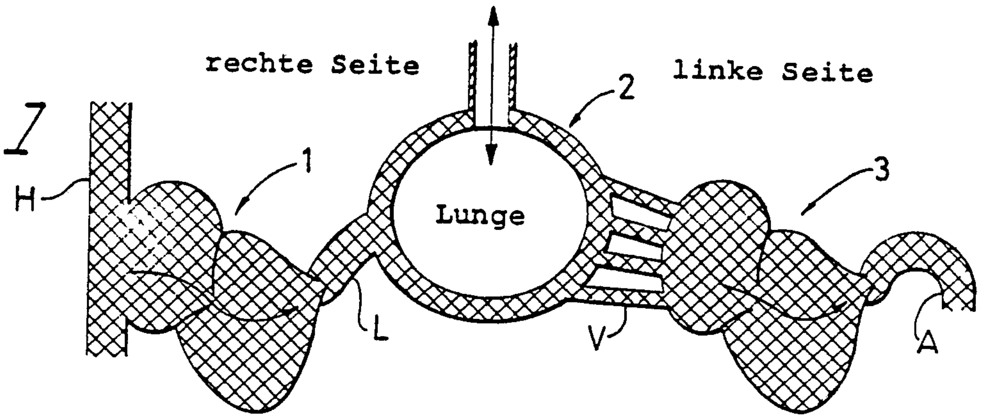


Fig. 2

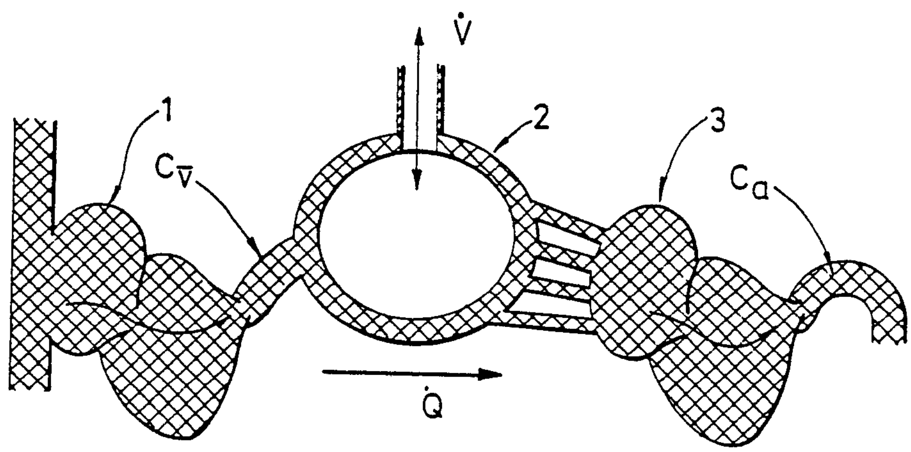


Fig. 3

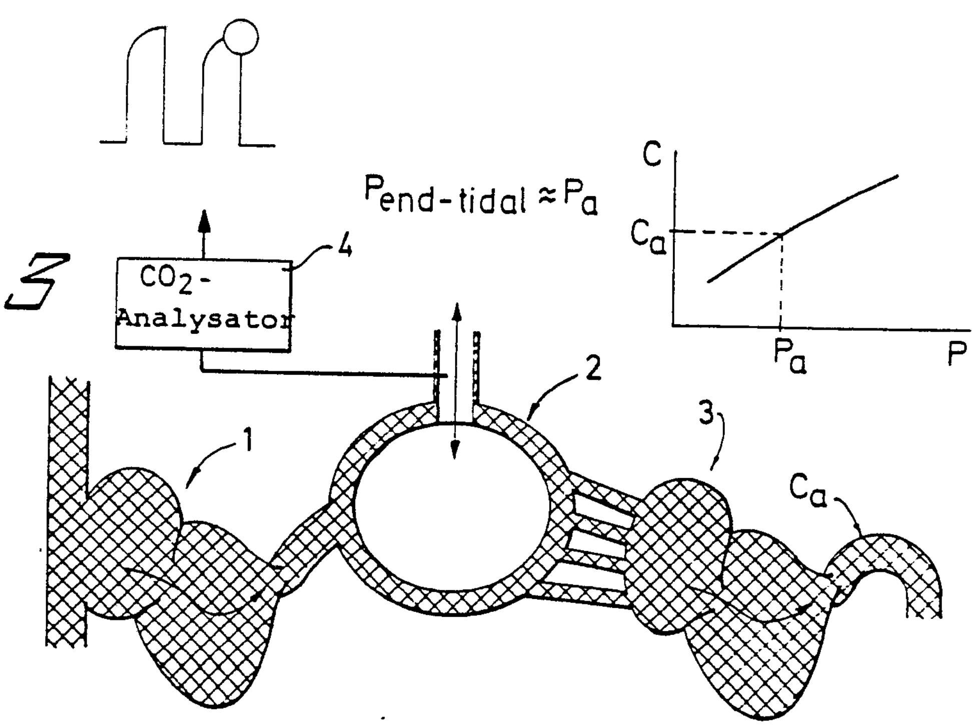

Fig. 4

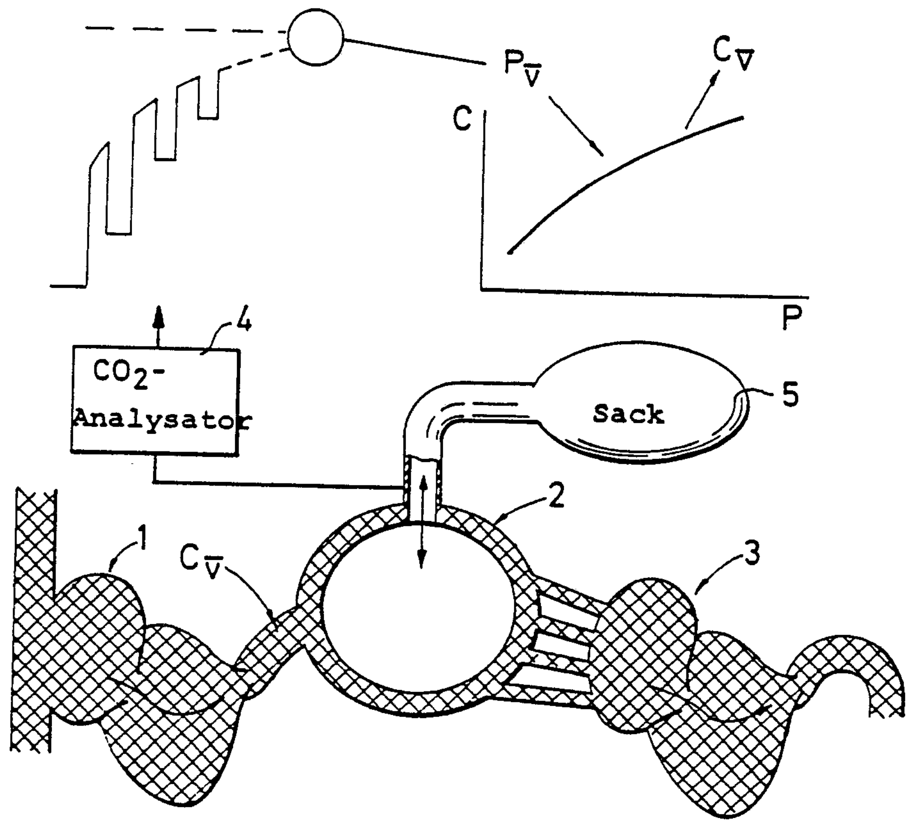

PV̄
CV̄
C
P
4
CO2-
Analysator
Sack
5
2
1
CV̄
3


Fig. 5

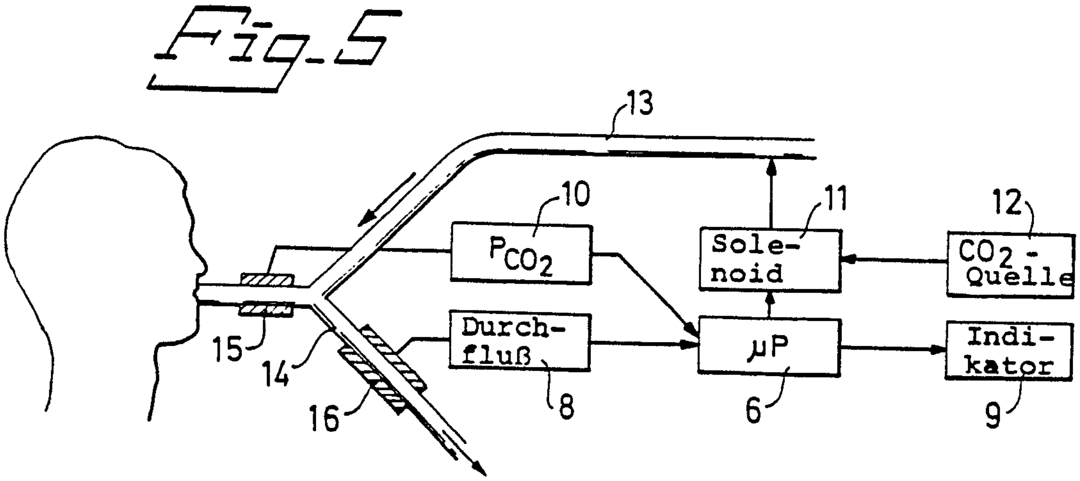

13
10
PCO2
11
Sole-
noid
12
CO2-
Quelle
Durch-
fluß
μP
Indi-
kator
15
14
16
8
6
9

Fig. 6

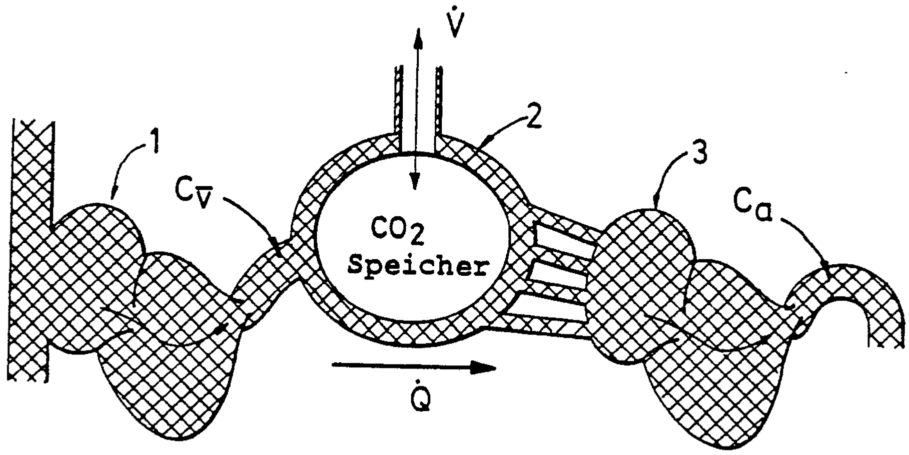
V̇
2
1
3
$C_{\bar{v}}$
$C_a$
$CO_2$
Speicher
Q̇

Fig. 7

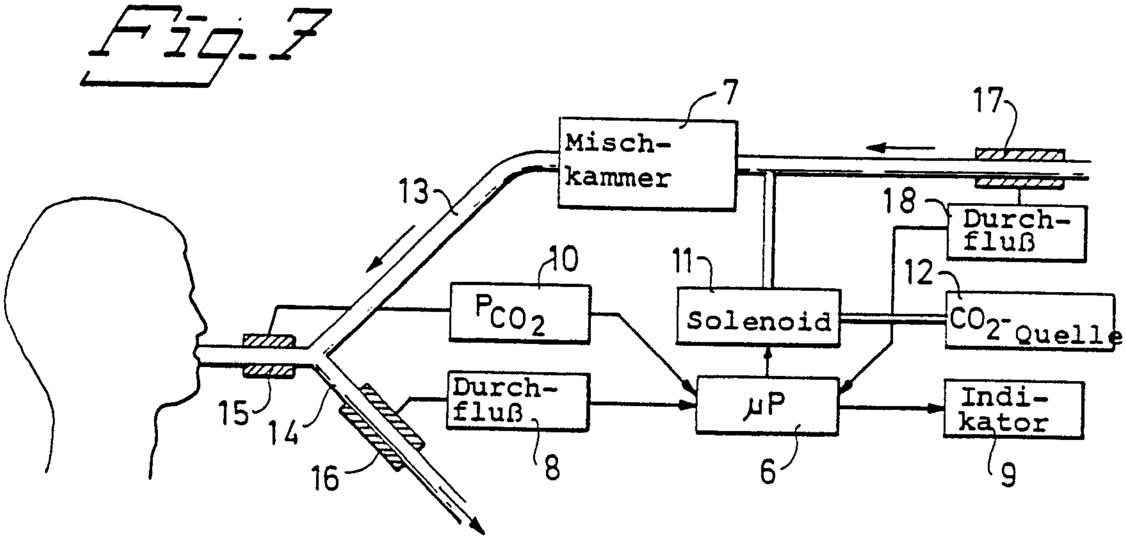
7
Misch-
kammer
17
13
18
Durch-
fluß
10
$P_{CO_2}$
11
Solenoid
12
$CO_2$-Quelle
15
14
16
Durch-
fluß
8
µP
6
Indi-
kator
9